(19) Europäisches Patentamt
European Patent Office
Office européen des brevets

(11) EP 2 111 842 A1

(12) # DEMANDE DE BREVET EUROPEEN

(43) Date de publication:
**28.10.2009 Bulletin 2009/44**

(21) Numéro de dépôt: **09156356.9**

(22) Date de dépôt: **26.03.2009**

(51) Int Cl.:
***A61K 8/19*** (2006.01) ***A61K 8/25*** (2006.01)
***A61K 8/41*** (2006.01) ***A61K 8/67*** (2006.01)
***A61K 8/68*** (2006.01) ***A61K 8/81*** (2006.01)
***A61K 8/84*** (2006.01) ***A61Q 5/10*** (2006.01)
***A61K 8/36*** (2006.01)

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **28.03.2008 FR 0852064**
**28.03.2008 FR 0852063**

(71) Demandeur: **L'Oréal**
**75008 Paris (FR)**

(72) Inventeurs:
- **Goget, Caroline**
  **75017, Paris (FR)**
- **Cottard, François**
  **92400, Courbevoie (FR)**
- **Laurent, Florence**
  **92270, Bois Colombes (FR)**
- **Ascione, Jean-Marc**
  **75003, Paris (FR)**

(74) Mandataire: **Wattremez, Catherine**
**L'OREAL**
**River Plaza - DIPI**
**25-29 Quai Aulagnier**
**92665 Asnières-sur-Seine (FR)**

(54) **Composition tinctoriale comprenant du chlorure d'ammonium, procédé de coloration de fibres kératiniques et dispositif**

(57) La présente invention a pour objet une composition tinctoriale comprenant, dans un milieu cosmétiquement acceptable, un ou plusieurs colorants d'oxydation, de l'ammoniaque, du chlorure d'ammonium et un ou plusieurs additifs choisis parmi les céramides, les silices, l'acide ascorbique et/ou ses sels, les alcanolamines et/ou leurs sels, les homopolymères d'acide acrylique réticulés, les copolymères de chlorure de dialkyldiallyl ammonium et d'acide acrylique ainsi que des polymères cationiques particuliers, ou leurs mélanges.

Elle concerne également un procédé de coloration de fibres kératiniques humaines mettant en oeuvre ladite composition en présence d'une composition comprenant au un ou plusieurs agents oxydants.

Un autre objet de l'invention concerne un dispositif à plusieurs compartiments approprié à la mise en oeuvre de ce procédé, comprenant dans un premier compartiment l'un d'eux une composition tinctoriale selon l'invention et dans un autre, une composition comprenant un ou plusieurs agents oxydants.

EP 2 111 842 A1

## Description

La présente invention a pour objet une composition comprenant, dans un milieu cosmétiquement acceptable, un ou plusieurs colorants d'oxydation, de l'ammoniaque, du chlorure d'ammonium et au moins un additif particulier. Elle concerne également un procédé de coloration de fibres kératiniques humaines mettant en oeuvre ladite composition ainsi qu'un dispositif à plusieurs compartiments approprié à la mise en oeuvre de ce procédé.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, plus particulièrement une ou plusieurs bases d'oxydation éventuellement associée(s) à un ou plusieurs coupleurs.

Habituellement, les bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Le procédé de coloration consiste à mettre en contact le ou les colorants d'oxydation avec un agent oxydant, qui est de préférence le peroxyde d'hydrogène, dans des conditions alcalines.

Pendant le temps de pause de la composition tinctoriale en présence de l'agent oxydant, deux phénomènes se produisent. L'un deux consiste en un éclaircissement des fibres kératiniques par l'action combinée de l'agent alcalin et de l'agent oxydant ; et l'autre en la réaction de condensation oxydative se produisant après diffusion des colorants à l'intérieur de la fibre, et conduisant à l'espèce colorée.

Traditionnellement, le temps de pause est de l'ordre d'une trentaine de minutes mais la tendance actuelle est de considérer qu'une telle durée est trop longue.

Pour résoudre ce problème, on ne peut raisonnablement envisager de simplement diminuer le temps de pause des compositions classiques. En effet, il est clair qu'il serait difficile de conserver le même niveau d'efficacité de telles compositions, en particulier de maintenir un niveau d'éclaircissement et de coloration satisfaisants, si elles étaient employées dans ces conditions raccourcies de durée.

Par ailleurs, on pourrait envisager d'augmenter la puissance de l'agent oxydant et de l'agent alcalin, par exemple en employant des agents oxydants du type des persels et/ou encore en augmentant le pH de la composition appliquée. Mais une telle option n'est peu souhaitable en ce sens que les moyens mis en oeuvre augmenteraient le risque de dégradation de la fibre kératinique.

Il existe donc un réel besoin de pouvoir disposer de compositions tinctoriales mettant en jeu des colorants d'oxydation, qui permettent de limiter la dégradation des fibres kératiniques traitées tout en conservant, voire en améliorant, les niveaux de coloration et d'éclaircissement par rapport à ceux obtenus avec les procédés classiques, en des temps de pause qui peuvent être avantageusement de l'ordre de 20 minutes au maximum, et plus particulièrement autour d'une quinzaine de minutes.

Ces buts et d'autres sont atteints par la présente invention qui a pour objet une composition de coloration des fibres kératiniques humaines, comprenant dans un milieu cosmétiquement acceptable, un ou plusieurs colorants d'oxydation, de l'ammoniaque, ainsi que du chlorure d'ammonium et un ou plusieurs additifs choisis parmi :

- les polymères cationiques constitués de motifs récurrents de formule suivante A:

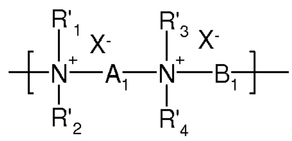

dans laquelle $R'_1$, $R'_2$, $R'_3$ et $R'_4$, identiques ou différents, représentent des groupes aliphatiques, alicycliques, ou aryle aliphatiques comprenant de 1 à 6 atomes de carbone ou des groupes hydroxy($C_1$-$C_6$)alkyle aliphatiques, ou bien $R'_1$, $R'_2$, $R'_3$ et $R'_4$, ensemble ou séparément, constituent avec les atomes d'azote auxquels ils sont rattachés des hétérocycles saturé à 5 ou 6 chaînons, comprenant éventuellement un second hétéroatome autre que l'azote (par exemple l'oxygène ou le soufre), ou bien $R'_1$, $R'_2$, $R'_3$ et $R'_4$ représentent un groupe alkyle en $C_1$-$C_6$, linéaire ou ramifié, substitué par un groupement nitrile, ester, acyle, amide ou -CO-O-$R'_5$-D ou -CO-NH-$R'_5$-D où $R'_5$ est un groupe alkylène en $C_1$-$C_6$ et D

un groupement ammonium quaternaire comprenant des groupements alkyle en $C_1$-$C_6$ identiques ou non ; $A_1$ et $B_1$ représentent des groupements polyméthyléniques comprenant de 2 à 6 atomes de carbone, pouvant être linéaires ou ramifiés, saturés ou insaturés, et pouvant contenir, liés à (substituant) ou intercalés dans la chaîne principale, un cycle aromatique en $C_6$, ou un atome d'oxygène, de soufre, des groupements sulfoxyde, sulfone, disulfure, amino, alkylamino, hydroxyle, ammonium quaternaire, uréido, ou ester, ou leurs combinaisons, et
$X^-$ désigne un anion dérivé d'un acide minéral ou organique ;
$A_1$, $R'_1$ et $R'_3$ peuvent former avec les deux atomes d'azote auxquels ils sont rattachés un cycle pipérazinique ; et les mélanges de ces additifs,

- les copolymères de chlorure de dialkyldiallyl ammonium et d'acide acrylique,
- les homopolymères d'acide acrylique réticulés,
- les céramides,
- les silices,
- l'acide ascorbique et/ou ses sels,
- les alcanolamines et/ou leurs sels.

Un autre objet de l'invention est constitué par un procédé de coloration de fibres kératiniques humaines dans lequel on applique la composition tinctoriale précédemment décrite, en présence d'un agent oxydant.
Enfin, l'invention concerne un dispositif à plusieurs compartiments comprenant dans un premier compartiment, une composition telle que définie précédemment et dépourvue d'agent oxydant, et dans un autre compartiment, une composition comprenant un ou plusieurs agents oxydants.
On a en effet remarqué que la composition selon l'invention permettait d'améliorer la puissance, la montée ainsi que l'homogénéité de la coloration obtenue, même avec des temps de pause de la composition de l'ordre d'une vingtaine de minutes au plus.
D'autres caractéristiques et avantages de l'invention apparaîtront plus clairement à la lecture de la description et des exemples.
Il est à noter que dans ce qui va suivre, et à moins d'une autre indication, les bornes d'un domaine de valeurs sont comprises dans ce domaine.
De plus, les fibres kératiniques humaines traitées par le procédé selon l'invention sont de préférence les cheveux.
Comme indiqué précédemment, la composition selon l'invention comprend du chlorure d'ammonium.
De préférence, le chlorure d'ammonium est présent dans la composition à une teneur supérieure à 1% en poids par rapport au poids de la composition.
Selon un mode de réalisation plus particulier de l'invention, la teneur en chlorure d'ammonium est d'au moins 1,5 % en poids et de préférence d'au moins 3 % en poids par rapport au poids de la composition.
Avantageusement, la teneur en chlorure d'ammonium représente entre 1 et 15% en poids, plus particulièrement entre 1,5 et 10% en poids, et de préférence entre 3 et 7% en poids, par rapport au poids de composition.
La composition tinctoriale selon l'invention comprend également de l'ammoniaque. Plus particulièrement, la teneur en ammoniaque dans la composition, exprimée en ammoniac gazeux, est d'au moins 0,5% en poids par rapport au poids de la composition.
Selon une variante avantageuse de l'invention, la teneur en ammoniaque, exprimée en ammoniac gazeux, est comprise 0,5 et 4% en poids, plus particulièrement entre 0,7 et 2,5% en poids, et de préférence entre 1 et 2% en poids, par rapport au poids de la composition.
Conformément à un mode de réalisation préféré de l'invention, les teneurs respectives de chlorure d'ammonium et d'ammoniaque dans la composition sont telles que le rapport pondéral de chlorure d'ammonium / ammoniaque (exprimé en ammoniac gazeux) est d'au moins 2. Selon une variante encore plus préférée, le rapport pondéral est compris 2 et 10, mieux entre 2,3 et 8 et de préférence entre 2,5 et 5.
La composition selon l'invention comprend également un ou plusieurs colorants d'oxydation. Plus particulièrement, le ou les colorants d'oxydation correspondent à une ou plusieurs bases d'oxydation, éventuellement associée (s) à un ou plusieurs coupleurs.
A titre d'exemples de bases d'oxydation, celles-ci sont choisies parmi les paraphénylènediamines, les bisphénylalkylènediamines, les para-aminophénols, les ortho-aminophénols, les bases hétérocycliques et leurs sels d'addition.
Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline,

la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de dérivés pyrazoliques on peut également citer les diamino N,N-dihydropyrazolopyrazolones et notamment celles décrites dans la demande FR 2886136 telles que les composés suivants et leurs sels d'addition : 2,3-Diamino-6,7-dihydro-1 H,5H-pyrazolo[1,2-a]pyrazol-1-one, 2-Amino-3-éthylamino-6,7-dihydro-1H,5H-pyrazolo[1,2-a]pyrazol-1-one, 2-Amino-3-isopropylamino-6,7-dihydro-1H,5H-pyrazolo[1,2-a]pyrazol-1-one, 2-Amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-Amino-1,2-diethyl-5-(pyrrolidin-1-yl)-1,2-dihydro-pyrazol-3-one, 4-Amino-5-(3-dimethylamino-pyrrolidin-1-yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques on utilisera préférentiellement le 4,5-diamino 1-(β-hydroxyéthyl) pyrazole et/ou la 2,3-diamino-6,7-dihydro-1H,5H-pyrazolo[1,2,A]pyrazol-1-one et leurs sels d'addition.

En ce qui concerne les coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemples, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthyiamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent avantageusement de 0,0001 à 10% en poids par rapport au poids de la composition, et de préférence de 0,005 à 5% en poids par rapport au poids de la composition.

La teneur en coupleur(s), s'il(s) est(sont) présent(s), représentent avantageusement de 0,0001 à 10% en poids par rapport au poids de la composition, et de préférence de 0,005 à 5 % en poids par rapport au poids de la composition.

Comme indiqué précédemment, la composition selon l'invention comprend un ou plusieurs additifs choisis les céramides, les silices, l'acide ascorbique et/ou ses sels, les alcanolamines et/ou leurs sels, les homopolymères d'acide acrylique réticulés, les copolymères de chlorure de dialkyldiallyl ammonium et d'acide acrylique, les polymères cationiques constitués de motifs récurrents de formule suivante A:

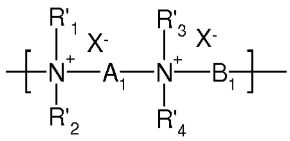

dans laquelle $R'_1$, $R'_2$, $R'_3$ et $R'_4$, identiques ou différents, représentent des groupes aliphatiques, alicycliques, ou aryle aliphatiques comprenant de 1 à 6 atomes de carbone
ou des groupes hydroxy($C_1$-$C_6$)alkyle aliphatiques, ou bien $R'_1$, $R'_2$, $R'_3$ et $R'_4$, ensemble
ou séparément, constituent avec les atomes d'azote auxquels ils sont rattachés des hétérocycles saturé à 5 ou 6 chaînons, comprenant éventuellement un second hétéroatome autre que l'azote (par exemple l'oxygène ou le soufre), ou bien $R'_1$, $R'_2$, $R'_3$ et $R'_4$ représentent un groupe alkyle en $C_1$-$C_6$, linéaire ou ramifié, substitué par un groupement nitrile, ester, acyle, amide ou -CO-O-$R'_5$-D ou -CO-NH-$R'_5$-D où $R'_5$ est un groupe alkylène en $C_1$-$C_6$ et D un groupement ammonium quaternaire comprenant des groupements alkyle en $C_1$-$C_6$ identiques ou non ;

$A_1$ et $B_1$ représentent des groupements polyméthyléniques comprenant de 2 à 6 atomes de carbone, pouvant être linéaires ou ramifiés, saturés ou insaturés, et pouvant contenir, liés à (substituant) ou intercalés dans la chaîne principale, un cycle aromatique en $C_6$, ou un atome d'oxygène, de soufre, des groupements sulfoxyde, sulfone, disulfure, amino, alkylamino, hydroxyle, ammonium quaternaire, uréido, ou ester, ou leurs combinaisons, et $X^-$ désigne un anion dérivé d'un acide minéral ou organique ;

$A_1$, $R'_1$ et $R'_3$ peuvent former avec les deux atomes d'azote auxquels ils sont rattachés un cycle pipérazinique ; et les mélanges de ces additifs.

La teneur en chacun des additifs précités représente plus particulièrement de 0,001 à 10% en poids, de préférence de 0,005% à 5% en poids, par rapport au poids de la composition.

Par céramide, on désigne en particulier des composés correspondant à la formule (B) suivante :

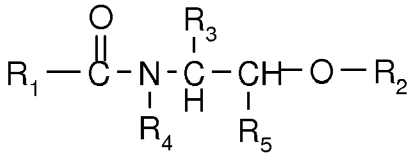

dans laquelle :

$R_1$ désigne :

- soit un radical hydrocarboné, linéaire ou ramifié, saturé ou insaturé, en $C_1$-$C_{50}$, de préférence en $C_5$-$C_{50}$, ce radical pouvant être substitué par un ou plusieurs et de préférence de un à six groupements hydroxyle éventuellement estérifiés par un acide $R_7COOH$, $R_7$ étant un radical hydrocarboné, saturé ou insaturé, linéaire ou ramifié, en $C_1$-$C_{35}$, éventuellement mono ou polyhydroxylé avec de préférence de un à six groupements hydroxyle, le ou les hydroxyles du radical $R_7$ pouvant être estérifiés par un acide gras saturé ou insaturé, linéaire ou ramifié en $C_1$-$C_{35}$, éventuellement mono ou polyhydroxylé avec de préférence de un à six groupements hydroxyle,
- soit un radical R''-(NR-CO)-R', R désigne un atome d'hydrogène ou un radical hydrocarboné, linéaire ou ramifié, saturé ou insaturé, $C_1$-$C_{20}$ mono ou polyhydroxylé avec de préférence de un à six groupements hydroxyle (préférentiellement monohydroxylé), R' et R'' sont des radicaux hydrocarbonés, linéaires ou ramifiés, saturés ou insaturés, dont la somme des atomes de carbone est comprise entre 9 et 30, R' étant un radical divalent,
- soit un radical $R_8$-O-CO-$(CH_2)_p$, $R_8$ désignant un radical hydrocarboné, linéaire ou ramifié, saturé ou insaturé, en $C_1$-$C_{20}$, p étant un entier variant de 1 à 12 ;

$R_2$ est choisi parmi un atome d'hydrogène, un radical de type saccharidique, en particulier un radical $(glycosyle)_n$, $(galactosyle)_m$ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;

$R_3$ désigne un atome d'hydrogène ou un radical hydrocarboné, linéaire ou ramifié, saturé ou insaturé, en $C_1$-$C_{33}$, hydroxylé avec de préférence de un à six groupements hydroxyle ou non hydroxylé, le ou les hydroxyle pouvant être estérifiés par un acide minéral ou un acide $R_7COOH$, $R_7$ ayant les mêmes significations que ci-dessus, le ou les hydroxyle pouvant être éthérifiés par un radical $(glycosyle)_n$, $(galactosyle)_m$, sulfogalactosyle, phosphoryléthylamine ou phosphoryléthyl ammonium, n est un entier variant de 1 à 4 et m est un entier variant de 1 à 8, $R_3$ pouvant également être substitué par un ou plusieurs radicaux alkyle en $C_1$-$C_{14}$ ;

$R_4$ désigne un atome d'hydrogène, un radical méthyle, éthyle, un radical hydrocarboné en $C_3$-$C_{50}$, saturé ou insaturé, linéaire ou ramifié, éventuellement hydroxylé ou un radical- $CH_2$-CHOH-$CH_2$-O-$R_6$ dans lequel $R_6$ désigne un radical hydrocarboné, linéaire ou ramifié, saturé ou insaturé, en $C_{10}$-$C_{26}$ ou un radical $R_8$-O-CO-$(CH_2)_p$, $R_8$ désigne un radical hydrocarboné, linéaire ou ramifié, saturé ou insaturé, en $C_1$-$C_{20}$, p étant un entier variant de 1 à 12,

$R_5$ désigne un atome d'hydrogène ou un radical hydrocarboné en $C_1$-$C_{30}$ saturé ou insaturé, linéaire ou ramifié, éventuellement mono ou polyhydroxylé avec de préférence de un à six groupements hydroxyle, le ou les hydroxyles pouvant être éthérifiés par un radical $(glycosyle)_n$, $(galactosyle)_m$, avec n représentant un entier variant de 1 à 4 et m un entier variant de 1 à 8, sulfogalactosyle, phosphoryléthylamine ou phosphoryléthylammonium,
sous réserve que lorsque $R_3$ et $R_5$ désignent hydrogène ou lorsque $R_3$ désigne hydrogène et $R_5$ désigne méthyle alors $R_4$ ne désigne pas un atome d'hydrogène, un radical méthyle ou éthyle.

Parmi les composés de formule (B) ci-dessus, on peut citer tout particulièrement les céramides et/ou glycocéramides décrits par DOWNING dans Journal of Lipid Research, Vol. 35, page 2060, 1994 ou ceux décrits dans la demande de brevet français FR-2 673 179.

Parmi les céramides préférés, on peut citer ceux pour lesquels, dans la formule (A), $R_1$ désigne un radical alkyle ou alcényle dérivé d'acides gras en $C_{14}$-$C_{22}$ éventuellement hydroxylé; $R_2$ désigne un atome d'hydrogène ; et $R_3$ désigne un radical linéaire saturé en $C_{11}$-$C_{17}$ éventuellement hydroxylé et de préférence en $C_{13}$-$C_{15}$.

De tels composés sont par exemple choisis parmi, seuls ou en mélange :

- la N-linoléoyldihydrosphingosine,
- la N-oléoyldihydrosphingosine ou 2-oléamido-1,3-octadécanediol,

- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
- le N-palmitamidohexadécanediol,

On peut aussi utiliser des mélanges spécifiques tels que par exemple les mélanges de céramide(s) 2 et de céramide(s) 5 selon la classification de DOWNING.

On peut également utiliser les composés de formule (B) pour lesquels $R_1$ désigne un radical alkyle ou alcényle dérivé d'acides gras en $C_{14}$-$C_{22}$; $R_2$ désigne un radical galactosyle ou sulfogalactosyle ; et $R_3$ désigne un radical hydrocarboné en $C_{12}$-$C_{22}$, saturé ou insaturé et de préférence un groupement $-CH=CH-(CH_2)_{12}-CH_3$.

Des composés de type céramide sont par exemple décrits dans les demandes de brevet DE 4424530, DE 4424533, DE 4402929, DE 4420736, WO 95/23807, WO 94/07844, EP 646572, WO 95/16665, FR 2673179, EP 227994 et WO 94/07844, WO 94/24097, WO 94/10131 auxquels on pourra se référer.

A titre d'exemples, on peut citer le produit constitué d'un mélange de glycocéramides, vendu sous la dénomination commerciale Glycocer® par la société Waitaki International Biosciences.

On peut également utiliser les composés décrits dans les demandes de brevet EP 227994, EP 647617, EP 736522 et WO 94/07844.

De tels composés sont par exemple le Questamide H®, encore appelé bis-(N-hydroxyéthyl N-cétyl) malonamide et vendu par la société Quest et le N-(2-hydroxyéthyl)-N-(3-cétyloxy-2-hydroxypropyl)amide d'acide cétylique .

On peut également utiliser le N-docosanoyl N-méthyl-D-glucamine tel que décrit dans la demande internationale WO 94/24097.

De préférence, si la composition comprend un ou plusieurs céramides, leur teneur représente de 0,001 à 1 % en poids, de préférence de 0,005 à 0,1% en poids, par rapport au poids de composition.

Conformément à une variante particulièrement avantageuse de l'invention, et lorsque la composition comprend un ou plusieurs céramides, le rapport massique céramide(s) / chlorure d'ammonium est de préférence compris entre 0,001 et 0,05.

Parmi les additifs possibles, on peut citer les silices. Ainsi, la composition peut comprendre plus particulièrement une ou plusieurs silices hydrophiles ou hydrophobes, ou leurs mélanges.

On entend par « silices hydrophiles » dans la présente invention aussi bien les silices hydrophiles pures que les particules totalement ou partiellement enrobées de silice hydrophile.

Les silices hydrophiles utilisables dans la composition de l'invention sont de préférence amorphes.

Elles se présentent généralement sous forme pulvérulente.

Elles peuvent en outre être d'origine pyrogénée ou d'origine précipitée.

Les silices pyrogénées sont obtenues habituellement par pyrolyse en flamme continue à 1000°C du tétrachlorure de silicium ($SiCl_4$) en présence d'hydrogène et d'oxygène.

Les silices précipitées sont obtenues plus particulièrement par réaction d'un acide sur des solutions de silicates alcalins, de préférence du silicate de sodium.

Selon un mode préféré de réalisation de l'invention, la silice hydrophile est choisie parmi les silices ayant une surface spécifique de 30 à 500 $m^2/g$, et une dimension moyenne de particules en nombre allant de 3 à 50 nm.

Ce sont plus particulièrement les silices hydrophiles décrites dans les tableaux (1) et (2) ci-dessous, et leurs mélanges.

Tableau (1)

| Nom commercial | AEROSIL 90 (Société Degussa-Hüls) | AEROSIL 130 (Société Degussa-Hüls) | AEROSIL 150 (Société Degussa-Hüls) | AEROSIL 200 (Société Degussa-Hüls) |
|---|---|---|---|---|
| Mode d'obtention | Pyrogénation | Pyrogénation | Pyrogénation | Pyrogénation |
| Surface BET ($m^2/g$) | $90 \pm 15$ | $130 \pm 25$ | $150 \pm 15$ | $200 \pm 15$ |
| Dimension moyenne des particules (nm) | 20 | 16 | 14 | 12 |
| Remarque | | | | taille agrégats : 10-30 $\mu m$ et 200 $\mu m$ |

Tableau (2)

| Nom commercial | AEROSIL 300 (Société Degussa-Hüls) | AEROSIL 380 (Société Degussa-Hüls) | AEROSIL OX 50 (Société Degussa-Hüls) | SILICE FK 320 DS (Société Degussa-Hüls) |
|---|---|---|---|---|
| Mode d'obtention | Pyrogénation | Pyrogénation | Pyrogénation | Précipitation |
| Surface BET ($m^2/g$) | 300 ± 30 | 380 ± 30 | 50 ± 25 | 170 ± 25 |
| Grandeur moyenne des particules (nm) | 7 | 7 | 40 | 18 |
| NB : le +/- donne le domaine de variation de la surface BET | | | | |

La silice hydrophile mise en oeuvre dans la composition selon l'invention peut consister également en une particule, notamment en une particule minérale, recouverte totalement ou partiellement de silice.

On peut citer en particulier les billes de silice contenant de l'oxyde de titane, commercialisées sous la dénomination TORAYCERAM S-IT® par la société Toray ; les micro-sphères de silice-alumine contenant de l'oxyde de titane (taille : 105 μm), commercialisées sous la dénomination Z-LIGHT-SPHERE W 1012® par la société Zeelan ; les particules de silice synthétique précipitée amorphe/oxyde de titane (taille : 106-500 μm), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 μm), commercialisée sous la dénomination ACS-0050510® par la société SACI-CFPA ; le nano-oxyde de titane anatase traité alumine et silice à 40% dans l'eau (taille : 60 nm, monodisperse), commercialisé sous la dénomination MIRASUN TIW 60® par la société Rhodia Chimie ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane : 30 nm ; épaisseur de silice : 2 nm) commercialisé sous la dénomination MAXLIGHT TS-042® par la société Nichimen Europe PLC.

On utilise de préférence comme silice hydrophile, les silices pyrogénées et en particulier celles commercialisées sous les dénominations AEROSIL (nom INCI : silica) et notamment celle commercialisée sous la dénomination AEROSIL 200 par la société Degussa-Hüls.

On entend par « silices hydrophobes » dans la présente invention aussi bien les silices hydrophobes pures que les particules totalement ou partiellement enrobées de silice hydrophobe.

Les silices hydrophobes pouvant être utilisées dans la composition de l'invention sont de préférence amorphes et d'origine pyrogénée.

Elles se présentent de préférence sous forme pulvérulente.

Les silices hydrophobes amorphes d'origine pyrogénée sont obtenues plus particulièrement à partir de silices hydrophiles. Ces dernières sont obtenues par pyrolyse en flamme continue à 1000°C du tétrachlorure de silicium ($SiCl_4$) en présence d'hydrogène et d'oxygène. Elles sont ensuite rendues hydrophobes par un traitement avec des silanes halogénés, des alcoxysilanes ou des silazanes. Les silices hydrophobes diffèrent des silices hydrophiles de départ par, entre autres, une densité de groupe silanols plus faible et par une adsorption de vapeur d'eau plus petite.

Selon un mode préféré de réalisation de l'invention, la silice hydrophobe est choisie parmi les silices ayant une surface spécifique de 50 à 500 $m^2/g$, et une dimension moyenne de particules en nombre allant de 3 à 50 nm.

Ce sont plus particulièrement les silices hydrophobes décrites dans le tableau (3) ci-dessous, et leurs mélanges.

Tableau (3)

| Nom commercial | AEROSIL R202 (Société DegussaHüls) | AEROSIL R805 (Société DegussaHüls) | AEROSIL R812 (Société DegussaHüls) | AEROSIL R972 (Société DegussaHüls) | AEROSIL R974 (Société DegussaHüls) |
|---|---|---|---|---|---|
| Surface BET ($m^2/g$) | 90 ± 20 | 150 ± 25 | 260 ± 30 | 110 ± 20 | 170 ± 20 |

(suite)

| Nom commercial | AEROSIL R202 (Société DegussaHüls) | AEROSIL R805 (Société DegussaHüls) | AEROSIL R812 (Société DegussaHüls) | AEROSIL R972 (Société DegussaHüls) | AEROSIL R974 (Société DegussaHüls) |
|---|---|---|---|---|---|
| Dimension moyenne des particules (nm) | 14 | 12 | 7 | 16 | 12 |

La silice hydrophobe utilisée dans la composition selon l'invention peut consister également en une particule, notamment minérale, telle que les pigments et oxydes métalliques, recouverts totalement ou partiellement de silice hydrophobe.

Ces particules peuvent aussi avoir des propriétés optiques dans le produit comme sur les cheveux, par exemple elles peuvent avoir un effet matifiant ou légèrement blanchissant.

On utilise de préférence comme silice hydrophobe, une silice pyrogénée hydrophobe traitée en surface par un diméthylsiloxane, comme celle commercialisée sous la dénomination AEROSIL R972 (nom INCI: Silica Dimethyl Silylate) par la société Degussa-Hüls.

De préférence les silices utilisées dans l'invention sont des silices hydrophobes.

De préférence, si la composition comprend de la silice, sa teneur représente de 0,1 à 10% en poids, plus particulièrement de 0,5 à 5% en poids, et encore plus préférentiellement de 1 à 3% en poids, par rapport au poids de composition.

Conformément à une variante particulièrement avantageuse de l'invention, et lorsque la composition comprend de la silice, le rapport massique silice / chlorure d'ammonium est de préférence compris entre 0,01 et 2, avantageusement entre 0,1 et 1 et encore plus préférentiellement entre 0,2 et 0,8.

Parmi les additifs envisageables, figurent l'acide ascorbique et ses sels, comme par exemple des sels de métaux alcalins comme le sodium, le potassium, de métaux alcalino-terreux comme le calcium, d'ammonium, d'amines primaires, secondaires ou tertiaires comprenant un à trois groupements identiques ou non de type alkyle en $C_1$-$C_4$ éventuellement porteur d'un groupement hydroxyle.

De préférence, si la composition comprend l'acide ascorbique, sous forme acide et/ou de sel, sa teneur exprimée sous forme acide représente de 0,01 à 10% en poids, plus particulièrement de 0,05 à 5% en poids, et de préférence de 0,1 à 1% en poids, par rapport au poids de composition.

Conformément à une variante particulièrement avantageuse de l'invention, et lorsque la composition comprend de l'acide ascorbique, sous forme acide et/ou de sel, le rapport massique acide ascorbique (exprimé sous forme acide) / chlorure d'ammonium est de préférence compris entre 0,5 et 0,005 et encore plus préférentiellement compris entre 0,2 et 0,01.

La composition selon l'invention peut éventuellement comprendre une ou plusieurs alcanolamines comme additifs.

Par alcanolamine au sens de la présente invention on entend un composé comportant une chaîne hydrocarbonée, linéaire ou ramifiée porteuse d'un ou plusieurs groupement hydroxyle et d'un ou plusieurs groupements amino ces derniers pouvant être éventuellement substitués

De préférence les alcanolamines sont de structure (C)

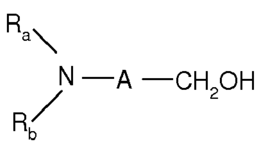

$R_a$ et $R_b$ désignant indépendamment l'un de l'autre un atome d'hydrogène, un radical alkyle linéaire ou ramifié en $C_1$-$C_{10}$ et de préférence en $C_1$-$C_4$, un radical mono ou polyhydroxyalkyle linéaire ou ramifié en $C_1$-$C_{10}$ et de préférence en $C_1C_4$ ;

A désignant un radical alkylène, linéaire ou ramifié en $C_1$-$C_{10}$, de préférence en $C_1$-$C_4$ éventuellement substitué par un ou plusieurs radicaux hydroxyle.

A titre d'alcanolamines on peut citer la monoéthanolamine, la diéthanolamine, la triéthanolamine, la monoisopropanolamine, la diisopropanolamine, la triisopropanolamine, la NNdiméthyléthanolamine,la NNdiéthyléthanolamine, le 2-amino 2-méthyl 1 propanol, le 2-amino 2-méthyl 1,3-propanediol, le 2-amino 2-hydroxyméthyl 1,3-propanediol.

De préférence l'alcanolamine est la monoéthanolamine.

A noter que l'alcanolamine peut se présenter sous forme libre ou sous la forme d'un sel avec un composé acide, comme par exemple les acides gras, l'acide ascorbique précités, l'acide polyacrylique réticulé détaillé plus bas, ainsi que les acides chlorhydrique, bromhydrique, sulfurique, phosphorique, acétique, citrique, succinique, tartrique, lactique, toluènesulfonique, benzènesulfonique.

En particulier, si la composition comprend une ou plusieurs alcanolamines, leur teneur totale, exprimée sous forme libre, représente de 0,1 à 15% en poids, plus particulièrement de 0,5 à 12% en poids, et de préférence de 1 à 10% en poids, par rapport au poids de composition.

Conformément à une variante préférée de l'invention, et lorsque la composition comprend une ou plusieurs alcanolamines, la teneur en chlorure d'ammonium est comprise entre 2, et 5% en poids, par rapport au poids de la composition.

En outre, selon cette variante, la teneur en ammoniaque, exprimée en ammoniac gazeux, est comprise entre 0,5 et 2 % en poids, par rapport au poids de la composition.

Conformément à une variante particulièrement avantageuse de l'invention, et lorsque la composition comprend une ou plusieurs alcanolamines, le rapport massique alcanolamines (exprimé sous forme non salifiée)/chlorure d'ammonium est de préférence compris entre 0,01 et 5, plus particulièrement entre 0,05 et 1, et encore plus préférentiellement entre 0,1 et 0.5.

La composition selon l'invention peut comprendre à titre d'additif, un ou plusieurs homopolymères d'acide acrylique réticulés, sous forme libre ou sous forme de sels, comme par exemple des sels de métaux alcalins comme le sodium, le potassium, de métaux alcalino-terreux comme le calcium, d'ammonium, d'amines primaires, secondaires ou tertiaires comprenant un à trois groupements identiques ou non de type alkyle en $C_1$-$C_4$ éventuellement porteur d'un groupement hydroxyle, de préférence de monoéthanolamine.

Il peut être réticulé en tout ou partie, par exemple avec un éther d'allyle.

Il peut s'agir par exemple d'un homopolymère d'acide acrylique réticulé avec un éther d'allyle et de pentaérythritol, avec un éther d'allyle et de sucrose, ou avec un éther d'allyle et de propylène. De tels polymères sont notamment classés sous la dénomination Carbomer dans le dans le dictionnaire CTFA, $9^{ème}$ édition, 2002. Ils sont entre autres commercialisés sous la dénomination CARBOPOL par la société NOVEON et sous la dénomination SYNTHALEN par la société 3V Ilnc.

Le poids moléculaire des homopolymères d'acide acrylique employés dans le cadre de l'invention, est plus particulièrement supérieur ou égal à 100 000 g/mol. De préférence, il est supérieur ou égal à 500 000 g/mol et de manière plus avantageuse, il est supérieur ou égal à 1 000 000 g/mol. Il est tout particulièrement compris entre 1 000 000 g/mol et 10 000 000 g/mol.

Avantageusement, si la composition comprend un ou plusieurs homopolymères d'acide acrylique, leur teneur, exprimée en acide polyacrylique, représente de 0,05 à 5% en poids, de façon plus avantageuse de 0,1 à 3% en poids, et de préférence de 0,2 à 1 % en poids, par rapport au poids de composition.

Conformément à une variante particulièrement avantageuse de l'invention, et lorsque la composition comprend un ou plusieurs homopolymères d'acide acrylique, le rapport massique homopolymères d'acide acrylique (exprimé en acide polyacrylique)/chlorure d'ammonium est de préférence compris entre 0,01 et 1, plus particulièrement entre 0,05 et 0,75, et encore plus préférentiellement entre 0,1 et 0,5.

La composition peut également comprendre un ou plusieurs copolymères de chlorure de dialkyldiallyl ammonium et d'acide acrylique.

En particulier, ledit polymère comprend au moins une unité de formule suivante

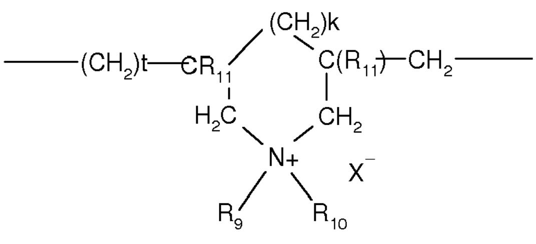

(D)

dans laquelle k et t sont égaux à 0 ou 1, la somme k + t étant égale à 1 ;

$R_9$ et $R_{10}$, identiques ou différents, désignent un groupement alkyle en $C_1$-$C_{26}$, un groupement hydroxyalkyle($C_1$-$C_5$), un groupement alkyl($C_1$-$C_2$)amidoalkyle($C_1$-$C_4$), ou $R_9$ et $R_{10}$ désignent conjointement avec l'atome d'azote auquel ils sont rattachés, un groupement pipéridinyle ou morpholinyle ;
$R_{11}$ désigne un atome d'hydrogène ou un radical méthyle ;
$X^-$ est un anion.

De préférence $R_9$ et $R_{10}$ désignent méthyle.

Ces polymères peuvent éventuellement comprendre dans leur structure des unités issues d'autres monomères comme par exemple l'acrylamide.

De tels polymères sont notamment classés sous les dénominations Polyquaternium-22 et Polyquaternium-39 dans le dans le dictionnaire CTFA, $9^{\text{ème}}$ édition, 2002.

Ils sont par exemple commercialisés par la société Ondeo Nalco sous les dénominations Merquat 295, Merquat 280 pour le Polyquaternium-22 et Merquat plus 3330 , Merquat Plus 3331 pour le Polyquaternium-39.

Avantageusement, si la composition comprend un ou plusieurs copolymères de ce type, leur teneur totale représente de 0,1 à 10 % en poids, plus particulièrement de 0,2 à 5, et de préférence de 0,5 à 2% en poids, par rapport au poids de composition.

Conformément à une variante particulièrement avantageuse de l'invention, et lorsque la composition comprend un ou plusieurs copolymères de chlorure de dialkyldiallyl ammonium et d'acide acrylique, le rapport massique copolymère / chlorure d'ammonium est de préférence compris entre 0,05 et 1, plus particulièrement entre 0,1 et 0,8 et encore plus préférentiellement entre 0,2 et 0,5.

La composition selon l'invention peut également comprendre un ou plusieurs polymères cationiques constitués de motifs récurrents de formule suivante (A)

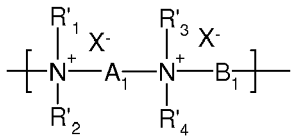

dans laquelle $R'_1$, $R'_2$, $R'_3$ et $R'_4$, identiques ou différents, représentent des groupes aliphatiques, alicycliques, ou aryle aliphatiques comprenant de 1 à 6 atomes de carbone ou des groupes hydroxy($C_1$-$C_6$)alkyle aliphatiques, ou bien $R'_1$, $R'_2$, $R'_3$ et $R'_4$, ensemble ou séparément, constituent avec les atomes d'azote auxquels ils sont rattachés des hétérocycles saturé à 5 ou 6 chaînons, comprenant éventuellement un second hétéroatome autre que l'azote (par exemple l'oxygène ou le soufre), ou bien $R'_1$, $R'_2$, $R'_3$ et $R'_4$ représentent un groupe alkyle en $C_1$-$C_6$, linéaire ou ramifié, substitué par un groupement nitrile, ester, acyle, amide ou -CO-O-$R'_5$-D ou -CO-NH-$R'_5$-D où $R'_5$ est un groupe alkylène en $C_1$-$C_6$ et D un groupement ammonium quaternaire comprenant des groupements alkyle en $C_1$-$C_6$ identiques ou non ;

$A_1$ et $B_1$ représentent des groupements polyméthyléniques comprenant de 2 à 6 atomes de carbone, pouvant être linéaires ou ramifiés, saturés ou insaturés, et pouvant contenir, liés à (substituant) ou intercalés dans la chaîne principale, un cycle aromatique en $C_6$, ou un atome d'oxygène, de soufre, des groupements sulfoxyde, sulfone, disulfure, amino, alkylamino, hydroxyle, ammonium quaternaire, uréido, ou ester, ou leurs combinaisons, et $X^-$ désigne un anion dérivé d'un acide minéral ou organique ;

$A_1$, $R'_1$ et $R'_3$ peuvent former avec les deux atomes d'azote auxquels ils sont rattachés un cycle pipérazinique.

Dans la formule précitée, de préférence, $X^-$ est un anion choisi parmi les ions bromure, chlorure, acétate, borate, citrate, tartrate, bisulfate, bisulfite, sulfate, phosphate, et de préférence chlorure bromure.

Des polymères de ce type sont notamment décrits dans les brevets français numéros 2 320 330, 2 270 846, 2 316 271, 2 336 434 et 2 413 907 et les brevets US numéros 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4.026.945 et 4 027 020.

On peut utiliser plus particulièrement les polymères qui sont constitués de motifs récurrents répondant à la formule :

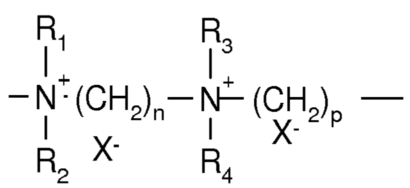

(a)

dans laquelle $R_1$, $R_2$, $R_3$ et $R_4$, identiques ou différents, désignent un groupe alkyle ou hydroxyalkyle ayant de 1 à 4 atomes de carbone environ, n et p sont des nombres entiers variant de 2 à 6 environ et, $X^-$ est un anion dérivé d'un acide minéral ou organique tel que défini plus haut.

Un composé de formule (a) particulièrement préféré est celui pour lequel $R_1$, $R_2$, $R_3$ et $R_4$ représentent un groupe méthyle et n = 3, p= 6 et X = Cl, c'est-à-dire le chlorure d'hexadimethrine dénommé "Hexadimethrine chloride"

selon la nomenclature INCI (CTFA).

Selon un mode de réalisation particulier, le polycondensat utile dans la présente invention présente une charge cationique supérieure à 5 meq/g, de préférence supérieure à 6 meq/g. Cette densité de charge peut se déterminer soit par calcul à partir de la structure du polymère soit expérimentalement par la méthode Kjeldahl.

Avantageusement, si la composition comprend un ou plusieurs polymères cationiques avec motifs récurrents (A), leur teneur totale représente de 0,1 à 10% en poids, plus particulièrement de 0,2 à 5, et de préférence de 0,5 à 3% en poids, par rapport au poids de composition.

Conformément à une variante particulièrement avantageuse de l'invention, et lorsque la composition comprend un ou plusieurs polymère de ce type, le rapport massique polymère cationique avec motifs récurrents (A)/chlorure d'ammonium est compris entre 0,05 et 10, plus particulièrement entre 0,1 et 5 et de préférence entre 0,2 et 1.

Selon un mode de réalisation plus particulier de l'invention, la composition comprend un ou plusieurs additifs choisis parmi les polymères cationiques constitués de motifs récurrents de formule A, les copolymères de chlorure de dialkyldiallyl ammonium et d'acide acrylique, les homopolymères d'acide acrylique réticulés, les céramides, les silices, et encore plus particulièrement les polymères cationiques constitués de motifs récurrents de formule A, les copolymères de chlorure de dialkyldiallyl ammonium et d'acide acrylique, les céramides.

De préférence les compositions de l'invention contiennent un ou plusieurs additifs choisis parmi les copolymères de chlorure de dialkyldiallyl ammonium et d'acide acrylique, les polymères cationiques constitués de motifs récurrents de formule A décrite ci-dessus.

La composition selon l'invention peut également comprendre un ou plusieurs acides gras.

Par acide gras, on désigne des composés comprenant au moins une fonction carboxylique sous forme libre et une chaîne hydrocarbonée, saturée ou insaturée, linéaire ou ramifiée, comportant de 8 à 40, de préférence de 8 à 30 atomes de carbone, éventuellement porteuse d'un ou plusieurs groupements hydroxyle, de préférence 1 ou 2 groupements hydroxyle. Si l'acide gras est insaturé, il peut comprendre notamment de une à quatre double-liaisons carbone-carbone, conjuguées ou non.

Parmi les acides gras possibles, on peut citer l'acide caprylique, laurique, myristique, palmitique, cétylique, stéarique, béhénique, arachidique, oléique, linoléique, linolénique, arachidonique, érucique, ou leurs mélanges, tel que l'acide cétylstéarylique.

De préférence, l'acide gras est un acide saturé.

Ces acides gras peuvent être salifiés. A titre de sels on peut notamment citer les sels de métaux alcalins comme le sodium, le potassium, de métaux alcalino-terreux comme le calcium, d'ammonium, d'amines primaires, secondaires ou tertiaires comprenant un à trois groupements identiques ou non de type alkyle en $C_1$-$C_4$ éventuellement porteur d'un groupement hydroxyle.

Avantageusement, si la composition comprend un ou plusieurs acides gras, sous forme d'acide et/ou de sel, leur teneur totale, exprimée sous forme acide, représente de 0,1 à 20% en poids, plus particulièrement de 0,5 à 15% en poids, et encore plus avantageusement de 1 à 10% en poids, par rapport au poids de composition.

Conformément à une variante particulièrement avantageuse de l'invention, et lorsque la composition comprend un ou plusieurs acides gras, le rapport massique acide gras (exprimé sous forme acide)/ chlorure d'ammonium est de préférence compris entre 0,01 et 10, plus particulièrement entre 0,1 et 5, et encore plus préférentiellement entre 0,2 et 2.

Selon un mode de réalisation particulier, la composition comprend un ou plusieurs agents tensioactifs. Ces derniers peuvent être choisis, seuls ou en mélanges, parmi des tensioactifs anioniques, amphotères, non ioniques, zwittérioniques et cationiques, et plus préférentiellement parmi des tensioactifs anioniques ou non ioniques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :

- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamidesulfosuccinates ;
- les alkylsulfoacétates ;
- les acylsarconisates ; et les acylglutamates :
- les esters d'alkyle et de polyglycoside-carboxylates tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les alkyl-D-galactoside uronates ;
- les alkyléther-carboxylates polyoxyalkylénés, les alkylaryléther-carboxylates polyoxyalkylénés, les alkylamidoéther carboxylates polyoxyalkylénés ;

  le groupe alkyle ou acyle (RCO-) de ces composés comportant de 10 à 24 atomes de carbone et le groupe aryle

désignant de préférence un groupe phényle ou benzyle ; le nombre de groupements oxyalkylénés, et de préférence oxyéthylénés, est compris entre 2 et 50.

De préférence, les tensioactifs anioniques, s'ils sont présents, sont choisis parmi les alkylsulfates, les alkyléthersulfates, les alkylamidoéthersulfates, les alkylsulfonates, les acyliséthionates, les N-acyltaurates, les alkyléthercarboxylates polyoxyalkylénés, les alkylamidoéther carboxylates polyoxyalkylénés ; le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :

- les alcools gras polyéthoxylés, polypropoxylés et/ou polyglycérolés,
- les alpha-diols polyéthoxylés, polypropoxylés et/ou polyglycérolés, le nombre de groupements oxyde d'éthylène ou oxyde de propylène allant de 2 à 50 ; le nombre de groupements glycérol allant de 2 à 30 ;
- les amides gras polyéthoxylés ayant de 2 à 30 moles d'oxyde d'éthylène ;
- les amides gras polyglycérolés comportant de 1 à 5 groupements glycérol ;
- les esters éthoxylés d'acides gras du sorbitane ayant de 2 à 30 moles d'oxyde d'éthylène, les esters d'acides gras du saccharose ;
- les alkylpolyglucosides, les dérivés de N-alkylglucamine ; ces composés comprenant au moins une chaîne grasse de type alkyle ou alcényle

ou au moins une chaîne alkyle ou alcényle comprenant 10 à 24 atomes de carbone.

- les copolymères d'oxyde d'éthylène et d'oxyde de propylène.

De préférence, les tensioactifs non ioniques, s'ils sont présents, sont choisis parmi les alcools gras polyéthoxylés ou polyglycérolés, les amides gras polyéthoxylés ayant de 2 à 30 moles d'oxyde d'éthylène ; les amides gras polyglycérolés comportant de 1 à 5 groupements glycérol ; ces composés comprenant au moins une chaîne grasse de type alkyle ou alcényle comprenant 10 à 24 atomes de carbone.

Habituellement, s'ils sont présents, le ou les agents tensioactifs représentent une quantité comprise entre 0,01 et 50% en poids, de préférence entre 0,1 et 25% en poids par rapport au poids de la composition.

La composition tinctoriale conforme à l'invention peut également renfermer divers adjuvants utilisés classiquement dans les compositions pour la teinture des cheveux, tels que par exemple des agent épaississants autres que les homopolymères d'acide acrylique réticulés, comme les épaississants cellulosiques (avec par exemple l'hydroxyéthycellulose, l'hydroxypropylcellulose, la carboxyméthylcellulose), la gomme de guar et ses dérivés (par exemple l'hydroxypropylguar), les gommes d'origine microbienne (notamment la gomme de xanthane, la gomme de scléroglucane) ; des agents épaississants minéraux comme notamment les argiles ; des agents antioxydants ou réducteurs différents de l'acide ascorbique tels que par exemple l'acide érythorbique, le sulfite, bisulfite ou métabisulfite d'ammonium, le thiolactate d'ammonium ; des agents de pénétration, des agents séquestrants comme l'éthylènediamine tétraacétique ou ses sels ; des parfums ; des oxydes de titane ; des tampons ; des agents dispersants ; des agents filmogènes autres que les polymères cationiques précédemment décrits ; des colorants directs synthétiques ou d'origine naturelle et des agents conservateurs.

Les adjuvants ci dessus sont en général présents en quantité comprise pour chacun d'eux entre 0,01 et 20% en poids par rapport au poids de la composition.

Le milieu cosmétiquement acceptable de la composition, qui est un milieu approprié pour la coloration des fibres kératiniques humaines, comprend de préférence de l'eau et un ou plusieurs solvants.

A titre d'exemples de tels solvants, on peut citer, les alcanols, linéaires ou ramifiés, en $C_2$-$C_4$, tels que l'éthanol et l'isopropanol ; les polyols et éthers de polyols comme le 2-butoxyéthanol, l'hexylène glycol, le propylèneglycol, le dipropylèneglycol, le monométhyléther de propylèneglycol, le monoéthyléther et le monométhyléther du diéthylèneglycol, le glycérol ainsi que les alcools aromatiques comme l'alcool benzylique, le benzyloxyéthanol ou le phénoxyéthanol, et leurs mélanges.

Le ou les solvants peuvent être présents dans des proportions allant de 1 à 40 % en poids par rapport au poids de la composition, et de préférence de 5 à 30 % en poids.

Le pH de la composition selon l'invention, dépourvue d'agent oxydant, est habituellement compris entre 6 et 11,5.

De manière particulièrement avantageuse, il est supérieur ou égal à 9,2. De préférence ce pH est compris entre 9,2 à 11, plus particulièrement entre 9,3 à 10 et encore plus préférentiellement entre 9,4 à 9,9.

Au sens de l'invention, préciser que la composition colorante (ou tinctoriale) ne comprend pas d'agent oxydant signifie qu'elle ne comprend pas d'agent oxydant chimique, et en particulier pas d'agent oxydant chimique choisi parmi

le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

Il peut être ajusté à la valeur désirée au moyen du chlorure d'ammonium et de l'ammoniaque et éventuellement d'un ou plusieurs agents acidifiants ou d'un ou plusieurs agents alcalinisants habituellement utilisés dans le domaine.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, les carbonates alcalins, les alcanolamines mentionnées auparavant, les hydroxydes de sodium ou de potassium et les composés de formule suivante :

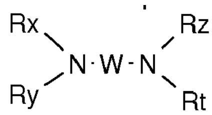

dans laquelle W est un reste propylène éventuellement substitué par un groupement hydroxyle ou un radical alkyle en $C_1$-$C_6$ ; Rx, Ry, Rz et Rt, identiques ou différents, représentent un atome d'hydrogène, un radical alkyle en $C_1$-$C_6$ ou hydroxyalkyle en $C_1$-$C_6$.

La composition selon l'invention peut être mélangée avec un ou plusieurs agents oxydants. On parle dans ce cas de composition prête à l'emploi.

En particulier, la composition prête à l'emploi est obtenue par mélange extemporané avant l'application, d'une composition dépourvue d'agent oxydant telle que précédemment décrite, avec au moins une composition comprenant un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Les compostions selon l'invention peuvent résulter du mélange extemporané de plusieurs compositions.

Un autre objet de l'invention est donc constitué par un procédé de coloration de fibres kératiniques humaines qui consiste à appliquer la composition décrite précédemment, en présence d'un ou plusieurs agents oxydants.

Selon une première variante de ce mode de réalisation, on applique sur les fibres la composition prête à l'emploi qui vient d'être détaillée et qui donc est obtenue par mélange extemporané avant l'application, d'une composition selon l'invention dépourvue d'agent oxydant avec une composition oxydante.

La composition colorante sans oxydant et la composition oxydante sont mélangées de préférence dans un rapport pondéral composition colorante sans oxydant/composition oxydante allant de 2 à 0,25 et de préférence de 1 à 0,5.

Le pH de la composition prête à l'emploi résultant du mélange va de préférence de 7,5 à 10, mieux de 8 à 9,8 et encore plus préférentiellement de 8,5 à 9,5.

Selon une deuxième variante de ce mode de réalisation, on applique la composition selon l'invention dépourvue d'agent oxydant, et une composition oxydante, successivement et sans rinçage intermédiaire.

La composition oxydante mise en oeuvre comprend un ou plusieurs agents oxydants tels que définis plus haut.

Concernant les solvants organiques éventuellement présents dans la composition oxydante, on pourra se reporter à la liste indiquée auparavant dans le cadre du descriptif de la composition selon l'invention.

Habituellement, le pH de la composition oxydante est inférieur à 7.

La composition oxydante peut se présenter sous la forme d'une solution, une émulsion ou un gel.

Elle peut éventuellement comprendre un ou plusieurs additifs utilisés classiquement dans le domaine de la coloration des fibres kératiniques humaines, en fonction de la forme galénique souhaitée. On pourra là encore se reporter à la liste des additifs donnée plus haut.

Quel que soit le mode de réalisation retenu (applications simultanée ou successive), le mélange appliqué sur les fibres est laissé en place pour une durée, en général, de l'ordre de 5 minutes à 60 minutes, de préférence de 5 minutes à 30 minutes, et encore plus avantageusement de 5 minutes à 20 minutes.

La température durant le procédé est classiquement comprise entre 10 et 200°C et plus particulièrement comprise entre la température ambiante (entre 15 à 25°C) et 80°C, de préférence entre la température ambiante et 60°C.

A l'issue du traitement, les fibres kératiniques humaines sont éventuellement rincées à l'eau, éventuellement

lavées au shampooing et rincées à nouveau à l'eau puis séchées ou laissées à sécher.

Un dernier objet de l'invention est constitué par un dispositif à plusieurs compartiments dont au moins l'un comprend la composition selon l'invention, dépourvue d'agent oxydant, et au moins un deuxième compartiment comprenant au moins une composition oxydante, comprenant un ou plusieurs agents oxydants.

Des exemples concrets mais non limitatifs de l'invention vont maintenant être présentés

## EXEMPLES

Dans les exemples qui vont suivre et à moins qu'une indication contraire ne soit donne, les quantités sont exprimées en % en poids de matière active.

### Exemple 1 :

Cet exemple illustre une composition selon l'invention comprenant une céramide en tant qu'additif.

On prépare la composition suivante :

| | |
|---|---|
| monoisopropanolamide d'acide laurique | 3 |
| alcool laurique oxyéthyléné (12 OE) | 7 |
| alcool oléocétylique oxyéthyléné (30 OE) | 4 |
| alcool décylique oxyéthyléné (3 OE) | 10 |
| alcool cétylstéarylique (C16-C18 - 50/50) | 11.5 |
| acide diéthylène triamine pentacétique, sel pentasodique en solution aqueuse à 40% | 4 |
| Kaolin | 1.2 |
| 1,3-diaminopropane | 0.4 |
| Polyquaternium 6 | 4 |
| 2-oléamido-1,3-octadécanediol (céramide) | 0.01 |
| propylène glycol | 7 |
| Carbopol ETD 2020 | 0.6 |
| chlorure d'ammonium | 3.78 |
| oxyde de titane | 0.15 |
| séquestrant | q.s.p |
| réducteurs | q.s.p |
| antioxydants | q.s.p |
| parfum | 0.6 |
| ammoniaque (à 20,5 % en ammoniac) | 6 |
| para-toluène diamine | 1.8 |
| 1-beta-hydroxyéthyloxy-2,4-diamino-benzène dichlorhydrate | 0.03 |
| 2-méthyl-1,3-dihydroxybenzène | 0.6 |
| méta-aminophénol | 0.4 |
| résorcinol | 1 |
| eau désionisée (qsp) | 100 |

On effectue ensuite le mélange de la composition avec une composition oxydante à 30 volumes (dilution 1 pour 1 en poids).

On applique le mélange résultant sur des mèches de cheveux naturels à 90 % de blancs.

On laisse pauser pendant 10 minutes à température ambiante (23°C+/-3°C).

A l'issue de ce temps de pause, les mèches sont rincées à l'eau, lavées avec un shampooing standard puis séchées.

On obtient ainsi une nuance châtain clair, avec une bonne qualité de la fibre

## Exemple 2

Cet exemple illustre une composition comprenant un polymère cationique particulier (Mexomere PO).

On prépare la composition suivante :

| | |
|---|---|
| monoisopropanolamide d'acide laurique | 3 |
| alcool laurique oxyéthyléné (12 OE) | 7 |
| alcool oléocétylique oxyéthyléné (30 OE) | 4 |
| alcool décylique oxyéthyléné (3 OE) | 10 |
| alcool cétylstéarilyque (C16-C18 - 50/50) | 11.5 |
| Kaolin | 1.2 |
| 1,3-diamino propane | 0.4 |
| acide diéthylène triamine pentacétique, sel pentasodique en solution aqueuse à 40 % | 4 |
| Mexomere PO | 3 |
| propylène glycol | 7 |
| acide polyacrylique réticulé | 0.6 |
| chlorure d'ammonium | 3.78 |
| oxyde de titane | 0.15 |
| séquestrant | q.s.p |
| réducteurs | q.s.p |
| antioxydants | q.s.p |
| parfum | 0.6 |
| ammoniaque (à 20.5 % en ammoniac) | 6 |
| sulfate de n,n-bis(2-hydroxyéthyl)-p-phénènediamine, 1 $H_2O$ | 0.25 |
| résorcinol | 1.3 |
| 1-hydroxy-3-amino-benzène | 0.3 |
| para-phénylènediamine | 1.8 |
| eau désionisée (qsp) | 100 |

On effectue ensuite le mélange de la composition avec une composition oxydante à 30 volumes (dilution 1 pour 1 en poids).

On applique le mélange résultant sur des mèches de cheveux naturels à 90 % de blancs.

On laisse pauser pendant 10 minutes à température ambiante (23°C+/-3°C).

A l'issue de ce temps de pause, les mèches sont rincées à l'eau, lavées avec un shampooing standard puis séchées.

On obtient ainsi une nuance châtain, avec une bonne qualité de la fibre.

## Exemple 3

Cet exemple illustre une composition comprenant de la silice.

On prépare la composition suivante :

| | |
|---|---|
| monoisopropanolamide d'acide laurique | 3 |

(suite)

| | |
|---|---|
| Alcool laurique oxyéthyléné (12 OE) | 7 |
| Alcool oléocétylique oxyéthyléné (30 OE) | 4 |
| Alcool décylique oxyéthyléné (3 OE) | 10 |
| Alcool cétylstéarilyque (C16-C18 - 50/50) | 11.5 |
| Silice pyrogénée à caractère hydrophobe | 1.2 |
| 1,3 diaminopropane | 0.4 |
| acide diéthylène triamine pentacétique, sel pentasodique en solution aqueuse à 40 % | 4 |
| Polyquaternium 6 | 4 |
| Propylène glycol | 7 |
| Carbopol ETD 2020 | 0.6 |
| Chlorure d'ammonium | 3.78 |
| Oxyde de titane | 0.15 |
| Séquestrants | q.s.p |
| Réducteurs | q.s.p |
| Antioxydants | q.s.p |
| Parfum | 0.6 |
| Ammoniaque (à 20.5 % en ammoniac) | 6 |
| Sulfate de n,n-bis(2-hydroxyethyl)-p-phenylenediamine, 1 h2o | 0.2 |
| Résorcinol | 1.3 |
| 1-hydroxy-3-amino-benzène | 0.3 |
| Para-phénylènediamine | 1.8 |
| Eau désionisée (qsp) | 100 |

On effectue ensuite le mélange de la composition avec une composition oxydante à 30 volumes (dilution 1 pour 1 en poids).
On applique le mélange résultant sur des mèches de cheveux naturels à 90 % de blancs.
On laisse pauser pendant 10 minutes à température ambiante (23°C+/-3°C).
A l'issue de ce temps de pause, les mèches sont rincées à l'eau, lavées avec un shampooing standard puis séchées.
On obtient ainsi une nuance châtain, avec une bonne qualité de la fibre.

**Exemple 4**

Cet exemple illustre une composition comprenant du Polyquaternium-22.
On prépare la composition suivante :

| | |
|---|---|
| monoisopropanolamide d'acide laurique | 3 |
| Alcool laurique oxyéthyléné (12 OE) | 7 |
| Alcool oléocétylique oxyéthyléné (30 OE) | 4 |
| Alcool décylique oxyéthyléné (3 OE) | 10 |
| Alcool cétylstéarilyque (C16-C18 - 50/50) | 11.5 |
| acide diéthylène triamine pentacétique, sel pentasodique en solution aqueuse à 40 % | 4 |
| Silice pyrogénée à caractère hydrophobe | 1.2 |

(suite)

| | |
|---|---|
| 1,3 diamino propane | 0.4 |
| Polyquaternium 22 | 3 |
| Propylène glycol | 7 |
| Carbopol ETD 2020 | 0.6 |
| Chlorure d'ammonium | 3.78 |
| Oxyde de titane | 0.15 |
| Séquestrants | q.s.p |
| Réducteurs | q.s.p |
| Antioxydants | q.s.p |
| Parfum | 0.6 |
| Ammoniaque (à 20.5 % en ammoniac) | 6 |
| para-toluène diamine | 1.1 |
| 2-méthylrésorcinol | 0.3 |
| 6-hydroxyindole | 0.03 |
| résorcinol | 0.6 |
| méta-aminophénol | 0.1 |
| para-aminophénol | 0.09 |
| Eau désionisée (qsp) | 100 |

On effectue ensuite le mélange de la composition avec une composition oxydante à 30 volumes (dilution 1 pour 1 en poids).
On applique le mélange résultant sur des mèches de cheveux naturels à 90 % de blanc.
On laisse pauser pendant 10 minutes à température ambiante (23°C+/-3°C).
A l'issue de ce temps de pause, les mèches sont rincées à l'eau, lavées avec un shampooing standard puis séchées.
On obtient ainsi une nuance châtain très clair, avec une bonne qualité de la fibre.

**Exemple 5**

Cet exemple illustre une composition comprenant un homopolymère d'acide acrylique réticulé.
On prépare la composition suivante :

| | |
|---|---|
| monoisopropanolamide d'acide laurique | 3 |
| Alcool laurique oxyéthyléné (12 OE) | 7 |
| Alcool oléocétylique oxyéthyléné (30 OE) | 4 |
| Alcool décylique oxyéthyléné (3 OE) | 10 |
| Alcool cétylstéarilyque (C16-C18 - 50/50) | 11.5 |
| acide diéthylène triamine pentacétique, sel pentasodique en solution aqueuse à 40 % | 4 |
| Kaolin | 1.2 |
| 1,3 diamino propane | 0.4 |
| Polyquaternium 6 | 4 |
| Propylène glycol | 7 |
| Acide polyacrylique réticulé (Carbomer 980) | 0.6 |

(suite)

| | |
|---|---|
| Chlorure d'ammonium | 3.78 |
| Oxyde de titane | 0.15 |
| Séquestrants | q.s.p |
| Réducteurs | q.s.p |
| Antioxydants | q.s.p |
| Parfum | 0.6 |
| Ammoniaque (à 20.5 % en ammoniac) | 6 |
| para-toluène diamine | 2 |
| 2,4-diaminophénoxyéthanol HCl | 0.02 |
| 2-méthylréorcinol | 0.76 |
| méta-aminophénol | 0.3 |
| résorcinol | 1. |
| para-aminophénol | 0.2 |
| eau désionisée (qsp) | 100 |

On effectue ensuite le mélange de la composition avec une composition oxydante à 30 volumes (dilution 1 pour 1 en poids).
On applique le mélange résultant sur des mèches de cheveux naturels à 90 % de blancs.
On laisse pauser pendant 10 minutes à température ambiante (23°C+/-3°C).
A l'issue de ce temps de pause, les mèches sont rincées à l'eau, lavées avec un shampooing standard puis séchées.
On obtient ainsi une nuance châtain doré, avec une bonne qualité de la fibre.

**Exemple 6**

Cet exemple illustre deux compositions comprenant de la monoéthanolamine.
On prépare les compositions suivantes :

| | | |
|---|---|---|
| monoisopropanolamide d'acide laurique | 3 | 3 |
| Alcool laurique oxyéthyléné (12 OE) | 7 | 7 |
| Alcool oléocétylique oxyéthyléné (30 OE) | 4 | 4 |
| Alcool décylique oxyéthyléné (3 OE) | 10 | 10 |
| Alcool cétylstéarilyque (C16-C18 - 50/50) | 11.5 | 11.5 |
| acide diéthylène triamine pentacétique, sel pentasodique en solution aqueuse à 40 % | 4 | 4 |
| Kaolin | 1.2 | 1.2 |
| Monoéthanolamine pure | 0.4 | 1.6 |
| Polyquaternium 6 | 4 | 4 |
| Propylène glycol | 7 | 7 |
| Carbopol ETD 2020 | 0.6 | 0.6 |
| Chlorure d'ammonium | 3.78 | 3.78 |
| Oxyde de titane | 0.15 | 0.15 |
| Séquestrants | q.s.p | q.s.p |
| Réducteurs | q.s.p | q.s.p |

(suite)

| | | |
|---|---|---|
| Antioxydants | q.s.p | q.s.p |
| Parfum | 0.6 | 0.6 |
| Ammoniaque (à 20.5 % en ammoniac) | 6 | 6 |
| para-toluène diamine | 0.9 | 0.9 |
| 2,4-diaminophénoxyéthanol HCl | 0.05 | 0.05 |
| 2-méthylrésorcinol | 0.6 | 0.6 |
| para-aminophénol | 0.8 | 0.8 |
| méta-aminophénol | 0.15 | 0.15 |
| résorcinol | 0.5 | 0.5 |
| 6-hydroxyindole | 0.15 | 0.15 |
| 2-méthyl-5-hydroxyéthylaminophénol | 0.3 | 0.3 |
| eau désionisée (qsp) | 100 | 100 |

On effectue ensuite le mélange de chaque composition avec une composition oxydante à 30 volumes (dilution 1 pour 1 en poids).

On applique chacun des mélanges résultants sur des mèches de cheveux naturels à 90% de blanc.

On laisse pauser pendant 10 minutes à température ambiante (23°C+/-3°C).

A l'issue de ce temps de pause, les mèches sont rincées à l'eau, lavées avec un shampooing standard puis séchées.

On obtient dans les deux cas une nuance cuivrée, avec une bonne qualité de la fibre.

**Exemple 7**

Cet exemple illustre une composition comprenant de l'acide ascorbique.

On prépare la composition suivante :

| | |
|---|---|
| monoisopropanolamide d'acide laurique | 3 |
| Alcool laurique oxyéthyléné (12 OE) | 7 |
| Alcool oléocétylique oxyéthyléné (30 OE) | 4 |
| Alcool décylique oxyéthyléné (3 OE) | 10 |
| Alcool cétylstéarilyque (C16-C18 - 50/50) | 11.5 |
| acide diéthylène triamine pentacétique, sel pentasodique en solution aqueuse à 40% | 4 |
| Kaolin | 1.2 |
| 1,3 diaminopropane | 0.4 |
| Polyquaternium 6 | 4 |
| Propylène glycol | 7 |
| Carbopol ETD 2020 | 0.6 |
| Chlorure d'ammonium | 3.78 |
| Oxyde de titane | 0.15 |
| Séquestrants | 0.25 |
| Acide ascorbique | 0.25 |
| Réducteurs | q.s.p |
| Parfum | 0.6 |

(suite)

| | |
|---|---|
| Ammoniaque (à 20.5 % en ammoniac) | 6 |
| para-toluène diamine | 0.9 |
| 2,4-diaminophénoxyéthanol HCl | 0.05 |
| 2-méthylrésorcinol | 0.6 |
| para-aminophénol | 0.8 |
| méta-aminophénol | 0.15 |
| résorcinol | 0.5 |
| 6-hydroxyindole | 0.15 |
| 2-méthyl-5-hydroxyéthylaminophénol | 0.3 |
| eau désionisée (qsp) | qsp 100 |

On effectue ensuite le mélange de la composition avec une composition oxydante à 30 volumes (dilution 1 pour 1 en poids).

On applique le mélange résultant sur des mèches de cheveux naturels à 90 % de blancs.

On laisse pauser pendant 10 minutes à température ambiante (23°C+/-3°C).

A l'issue de ce temps de pause, les mèches sont rincées à l'eau, lavées avec un shampooing standard puis séchées.

On obtient ainsi une nuance cuivrée avec une bonne qualité de la fibre.

## Exemple 8

Cet exemple illustre l'influence de la présence d'un polymère cationique sur les propriétés tinctoriales de la composition résultante.

On prépare les compositions suivantes (quantités données en g % de matière active) :

| | **Composition A invention** | **Composition B comparative** |
|---|---|---|
| Alcool laurique oxyéthyléné (120E) | 7.0 | 7.0 |
| Alcool oléocéthylique oxyéthyléné (30OE) | 4.0 | 4.0 |
| Alcool décylique oxyéthyléné (3OE) | 10 | 10 |
| Alcool cétylstéarilyque (C16-C18 - 50/50) | 11.5 | 11.5 |
| Polycondensat tétraméthyl hexaméthylènediamine/ dichloro 1,3- propylène en solution aqueuse | 2.4 | - |
| Propylène glycol | 7.0 | 7.0 |
| Chlorure d'ammonium | 3.78 | 3.78 |
| Oxyde de titane | 0.15 | 0.15 |
| Distéarate de glycol | 2.0 | 2.0 |
| Réducteur | qs | qs |
| Séquestrant | qs | qs |
| Parfum | qs | qs |
| Eau désionisée (QS) | QSP 100 | QSP 100 |
| Ammoniaque (à 20.5% en ammoniac) | 6 | 6 |
| Toluène-2,5- diamine | 0.37 | 0.37 |
| 1-méthyl-2-hydroxy-4-amino-benzène | 0.82 | 0.82 |
| para-aminophénol | 0.47 | 0.47 |
| 1,3-dihydroxybenzène (Résorcinol) | 0.01 | 0.01 |
| 1-méthyl-2-hydroxy-4-béta-hydroxyéthylamino-benzène | 0.615 | 0.615 |

Chaque composition est mélangée avec un oxydant à 30 volumes (dilution 1 pour 1 en poids).

On applique chacun des mélanges résultants sur des mèches de cheveux moyennement sensibilisés (solubilité alcaline 20 %).

On laisse pauser les mèches ainsi traitées pendant 10 minutes à température ambiante (23 °C+/-3 °C).

A l'issue de ce temps de pause, les mèches sont rincées à l'eau ; lavées avec un shampooing standard, puis séchées.

24 heures après coloration, on procède à des mesures colorimétriques à l'aide d'un colorimètre Minolta CM-2600D, en système Lab.

On mesure la puissance et la montée entre les compositions A et B :

**La puissance** est évaluée par la valeur de L*.

Plus L* est faible, plus la coloration obtenue est puissante.

**La montée** correspond à la différence de couleur entre la mèche non colorée et la mèche colorée.

On la calcule selon l'équation suivante :

$$DE = [\,(L^*-L^*_0)^2 + (a^* + a^*_0)^2 + (b^* - b^*_0)^2]^{1/2}$$

dans laquelle L*, a*, b* représentent les coefficients concernant la mèche colorée et $L^*_0$, $a^*_0$ et $b^*_0$ représentent les coefficients concernant la mèche non colorée.

Plus DE est élevée, plus la montée de la couleur est importante.

**Résultats**

| **Composition** | **L*** | **DE** |
|---|---|---|
| Mèche non colorée | 59.78 | - |
| Composition A selon l'invention | 22.64 | 43.76 |
| Composition B comparative | 26.05 | 40.24 |

On constate d'après ces résultats que la composition selon l'invention permet d'obtenir des colorations plus puissantes et avec une montée plus importante, que celles obtenues en mettant en oeuvre des compositions comparatives ne comprenant pas de polymère cationique.

## Revendications

1. Composition de coloration des fibres kératiniques comprenant dans un milieu cosmétiquement acceptable, un ou plusieurs colorants d'oxydation, de l'ammoniaque, du chlorure d'ammonium à une teneur supérieure à 1 % en poids par rapport au poids de la composition, et un ou plusieurs additifs choisis parmi :

   - les polymères cationiques constitués de motifs récurrents de formule suivante A:

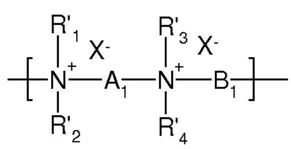

dans laquelle $R'_1$, $R'_2$, $R'_3$ et $R'_4$, identiques ou différents, représentent des groupes aliphatiques, alicycliques, ou aryle aliphatiques comprenant de 1 à 6 atomes de carbone ou des groupes hydroxy($C_1$-$C_6$)alkyle aliphatiques, ou bien $R'_1$, $R'_2$, $R'_3$ et $R'_4$, ensemble ou séparément, constituent avec les atomes d'azote auxquels ils sont rattachés des hétérocycles saturé à 5 ou 6 chaînons, comprenant éventuellement un second hétéroatome autre que l'azote (par exemple l'oxygène ou le soufre), ou bien $R'_1$, $R'_2$, $R'_3$ et $R'_4$ représentent un groupe alkyle en $C_1$-$C_6$, linéaire ou ramifié,

substitué par un groupement nitrile, ester, acyle, amide ou -CO-O-$R'_5$-D ou -CO-NH-$R'_5$-D où $R'_5$ est un groupe alkylène en $C_1$-$C_6$ et D un groupement ammonium quaternaire comprenant des groupements alkyle en $C_1$-$C_6$ identiques ou non ;
$A_1$ et $B_1$ représentent des groupements polyméthyléniques comprenant de 2 à 6 atomes de carbone, pouvant être linéaires ou ramifiés, saturés ou insaturés, et pouvant contenir, liés à (substituant) ou intercalés dans la chaîne principale, un cycle aromatique en $C_6$, ou un atome d'oxygène, de soufre, des groupements sulfoxyde, sulfone, disulfure, amino, alkylamino, hydroxyle, ammonium quaternaire, uréido, ou ester, ou leurs combinaisons, et
$X^-$ désigne un anion dérivé d'un acide minéral ou organique ;
$A_1$, $R'_1$ et $R'_3$ peuvent former avec les deux atomes d'azote auxquels ils sont rattachés un cycle pipérazinique ; et les mélanges de ces additifs

- les copolymères de chlorure de dialkyldiallyl ammonium et d'acide acrylique,
- les homopolymères d'acide acrylique réticulés,
- les céramides,
- les silices,
- l'acide ascorbique et/ou ses sels,
- les alcanolamines et/ou leurs sels.

**2.** Composition selon la revendication précédente, **caractérisée en ce que** la teneur en chlorure d'ammonium est d'au moins 1,5 % en poids par rapport au poids de la composition.

**3.** Composition selon l'une quelconque des revendications précédentes, **caractérisée en ce que** la teneur en chlorure d'ammonium est d'au moins 3 % en poids par rapport au poids de la composition.

**4.** Composition selon l'une quelconque des revendications précédentes, **caractérisée en ce que** la teneur en ammoniaque, exprimée en ammoniac gazeux, est d'au moins 0,5% en poids par rapport au poids de la composition.

**5.** Composition selon l'une quelconque des revendications précédentes, **caractérisée en ce que** le rapport pondéral de chlorure d'ammonium / ammoniaque (exprimé en ammoniac gazeux) est d'au moins 2.

**6.** Composition selon l'une quelconque des revendications précédentes **caractérisée en ce que** la teneur en chacun des additifs représente de 0,001 à 10% en poids, par rapport au poids de la composition.

**7.** Composition selon l'une quelconque des revendications précédentes, **caractérisée en ce que** le ou les additifs sont choisis parmi , les polymères cationiques constitués de motifs récurrents de formule A, les copolymères de chlorure de dialkyldiallyl ammonium et d'acide acrylique, les homopolymères d'acide acrylique réticulés, les céramides, les silices.

**8.** Composition selon l'une quelconque des revendications précédentes, **caractérisée en ce que** le ou les additifs sont choisis parmi les polymères cationiques constitués de motifs récurrents de formule A, les copolymères de chlorure de dialkyldiallyl ammonium et d'acide acrylique, les céramides.

**9.** Composition selon l'une quelconque des revendications précédentes, **caractérisée en ce qu'**elle comprend un ou plusieurs agents oxydants.

**10.** Composition selon la revendication précédente, **caractérisée en ce que** l'agent oxydant est le peroxyde d'hydrogène.

**11.** Composition selon l'une quelconque des revendications 1 à 8, **caractérisée en ce que** le pH de la composition est supérieur ou égal à 9,2, plus particulièrement compris entre 9,2 à 11.

**12.** Procédé de coloration des fibres kératiniques humaines, dans lequel on met en oeuvre une composition de coloration selon l'une quelconque des revendications 1 à 8 en présence d'une composition comprenant un ou plusieurs agents oxydants.

**13.** Procédé selon la revendication 12, **caractérisé en ce que** l'on applique sur les fibres kératiniques, successivement et sans rinçage intermédiaire, la composition de coloration puis une composition comprenant un ou plusieurs agents oxydants, ou l'inverse.

**14.** Procédé de coloration selon la revendication 12, **caractérisé en ce que** l'on met en oeuvre une composition prête à l'emploi, obtenue par mélange extemporané avant l'application, de la composition de coloration selon l'une des revendications 1 à 8 avec au moins une composition comprenant un ou plusieurs agents oxydants.

**15.** Dispositif à plusieurs compartiments comprenant dans un premier compartiment, une composition telle que définie dans l'une des revendications 1 à 8, et dans un autre compartiment, une composition comprenant un ou plusieurs agents oxydants.

Europäisches Patentamt
European Patent Office
Office européen des brevets

# RAPPORT DE RECHERCHE EUROPEENNE

Numéro de la demande
EP 09 15 6356

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2007/157399 A1 (NOBUTO YUKO [JP] ET AL) 12 juillet 2007 (2007-07-12)<br>Comparative ex. 2* exemples 4-6 *<br>----- | 1-15 | INV.<br>A61K8/19<br>A61K8/25<br>A61K8/41<br>A61K8/67<br>A61K8/68<br>A61K8/81<br>A61K8/84<br>A61Q5/10<br>A61K8/36 |
| E | WO 2009/050295 A (OREAL [FR]; LEGRAND FREDERIC [JP]; ASCIONE JEAN-MARC [FR]) 23 avril 2009 (2009-04-23)<br>* exemples A1,A3,A'1,A'3 *<br>----- | 1-15 | |
| A | EP 1 155 679 A (KAO CORP [JP]) 21 novembre 2001 (2001-11-21)<br>* exemples 1,2 *<br>----- | 1-15 | |
| A | US 5 334 225 A (OGAWA MASAHIKO [JP] ET AL) 2 août 1994 (1994-08-02)<br>* exemple 23 *<br>----- | 1-15 | |
| A | US 2002/088063 A1 (OHASHI YUKIHIRO [JP] ET AL) 11 juillet 2002 (2002-07-11)<br>* exemples 6-13 *<br>----- | 1-15 | |

DOMAINES TECHNIQUES RECHERCHES (IPC)

A61K

~~Le présent rapport a été établi pour toutes les revendications~~

2

| Lieu de la recherche | Date d'achèvement de la recherche | Examinateur |
|---|---|---|
| Munich | 1 juillet 2009 | Vayssié, Stéphane |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Europäisches Patentamt
European Patent Office
Office européen des brevets

Numéro de la demande

EP 09 15 6356

## REVENDICATIONS DONNANT LIEU AU PAIEMENT DE TAXES

La présente demande de brevet européen comportait lors de son dépôt les revendications dont le paiement était dû.

- [ ] Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

- [ ] Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

voir feuille supplémentaire B

- [ ] Toutes les nouvelles taxes de recherche ayant été acquittées dans les délais impartis, le présent rapport de recherche européenne a été établi pour toutes les revendications.

- [ ] Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

- [ ] Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

- [X] Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

  voir annexe

- [ ] Le present rapport supplémentaire de recherche européenne a été établi pour les parties de la demande de brevet européen qui se rapportent a l'invention mentionée en premier lieu dans le revendications (Règle 164 (1) CBE)

Europäisches Patentamt
European Patent Office
Office européen des brevets

**ABSENCE D'UNITÉ D'INVENTION**
**FEUILLE SUPPLÉMENTAIRE B**

Numéro de la demande
EP 09 15 6356

La division de la recherche estime que la présente demande de brevet européen ne satisfait pas à l'exigence relative à l'unité d'invention et concerne plusieurs inventions ou pluralités d'inventions, à savoir :

1. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les polymères cationiques de motifs récurrents A
ainsi qu'un procédé mettant en oeuvre cette composition (rev. 12) et un dispositif incorporant cette composition (rev. 15)

---

2. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les copolymères de chlorure de dialkyldiallyl ammonium et d'acide acrylique
ainsi qu'un procédé mettant en oeuvre cette composition (rev. 12) et un dispositif incorporant cette composition (rev. 15)

---

3. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les homopolymères d'acide acrylique réticulés
ainsi qu'un procédé mettant en oeuvre cette composition (rev. 12) et un dispositif incorporant cette composition (rev. 15)

---

4. revendications: 1-15 (partie)

EPO FORM P0402

Europäisches Patentamt
European Patent Office
Office européen des brevets

**ABSENCE D'UNITÉ D'INVENTION**
**FEUILLE SUPPLÉMENTAIRE B**

Numéro de la demande
EP 09 15 6356

La division de la recherche estime que la présente demande de brevet européen ne satisfait pas à l'exigence relative à l'unité d'invention et concerne plusieurs inventions ou pluralités d'inventions, à savoir :

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les céramides
ainsi qu'un procédé mettant en oeuvre cette composition (rev. 12) et un dispositif incorporant cette composition (rev. 15)

---

5. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les silices
ainsi qu'un procédé mettant en oeuvre cette composition (rev. 12) et un dispositif incorporant cette composition (rev. 15)

---

6. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi l'acide ascorbique et/ou ses sels
ainsi qu'un procédé mettant en oeuvre cette composition (rev. 12) et un dispositif incorporant cette composition (rev. 15)

---

7. revendications: 1-15 (partie)

composition de coloration des fibres kératiniques humaines comprenant dans un milieu cosmétiquement acceptable:
-un ou plusieurs colorants d'oxydation
-de l'ammoniaque
-du chlorure d'ammonium à une teneur supérieure à 1%
-un additif choisi parmi les alcanolamines et/ou leurs sels
ainsi qu'un procédé mettant en oeuvre cette composition (rev. 12) et un dispositif incorporant cette composition (rev. 15)

---

EPO FORM P0402

**ANNEXE AU RAPPORT DE RECHERCHE EUROPEENNE RELATIF A LA DEMANDE DE BREVET EUROPEEN NO.** EP 09 15 6356

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-07-2009

| Document brevet cité au rapport de recherche | Date de publication | Membre(s) de la famille de brevet(s) | Date de publication |
|---|---|---|---|
| US 2007157399 A1 | 12-07-2007 | EP 1803434 A1 | 04-07-2007 |
| | | JP 2007197418 A | 09-08-2007 |
| WO 2009050295 A | 23-04-2009 | FR 2922444 A1 | 24-04-2009 |
| EP 1155679 A | 21-11-2001 | DE 60126863 T2 | 20-12-2007 |
| | | JP 2001316232 A | 13-11-2001 |
| US 5334225 A | 02-08-1994 | EP 0579204 A2 | 19-01-1994 |
| US 2002088063 A1 | 11-07-2002 | DE 60123332 T2 | 16-05-2007 |
| | | EP 1166757 A2 | 02-01-2002 |
| | | JP 4139552 B2 | 27-08-2008 |
| | | JP 2002012530 A | 15-01-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136
- FR 2673179
- DE 4424530
- DE 4424533
- DE 4402929
- DE 4420736
- WO 9523807 A
- WO 9407844 A
- EP 646572 A
- WO 9516665 A
- EP 227994 A
- WO 9424097 A
- WO 9410131 A
- EP 647617 A
- EP 736522 A
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A

### Littérature non-brevet citée dans la description

- **DOWNING.** *Journal of Lipid Research,* 1994, vol. 35, 2060